Europäisches Patentamt

European Patent Office

Office européen des brevets

(19)

(11) Publication number: **0 159 602**
A2

(12) **EUROPEAN PATENT APPLICATION**

(21) Application number: 85104173.1

(22) Date of filing: 04.04.85

(51) Int. Cl.⁴: **A 61 K 33/34,** A 61 K 47/00,
A 61 K 9/22

(30) Priority: 11.04.84 AU 4528/84

(43) Date of publication of application: 30.10.85
Bulletin 85/44

(84) Designated Contracting States: **AT BE CH DE FR IT LI
LU NL SE**

(71) Applicant: **ICI AUSTRALIA LIMITED, 1 Nicholson Street,
Melbourne Victoria 3001 (AU)**

(72) Inventor: **Hamilton, Robert John, 22a Earl Street,
Boronia Park New South Wales 2111 (AU)**

(74) Representative: **Andrae, Steffen, Dr. et al,
Patentanwälte Andrae, Flach, Haug, Kneissl
Steinstrasse 44, D-8000 München 80 (DE)**

(54) Compositions and methods for the therapeutic treatment of trace element deficiencies in ruminant animals.

(57) A therapeutic composition for the treatment of trace element deficiency in ruminant animals wherein said composition comprises a particulate source of at least one trace element suspended in a matrix of solid polyethylene glycol.

EP 0 159 602 A2

0159602

Dipl.-Chem. Dr. Steffen ANDRAE
Dipl.-Phys. Dieter FLACH
Dipl.-Ing. Dietmar HAUG
Dipl.-Chem. Dr. Richard KNEISSL
PATENTANWÄLTE
Steinstr. 44, D-8000 München 80

0 4. APR. 1985

- 1 -

"COMPOSITION"

TITLE MODIFIED
see front page

This invention relates to compositions and methods for the therapeutic treatment of trace element deficiencies in ruminant animals.

The role of trace elements such as copper, iron, molybdenum, cobalt, nickel, zinc and manganese in ruminant nutrition is well known, and in particular the unusually large requirement of copper by sheep and cattle is well recognized.  Trace element deficiencies in ruminant animals frequently arise because the elements are not present or are present only in low quantities in the plants on which the animals are grazing, and this is commonly due to the lack of the particular trace elements in the soil.  In such cases the deficiency in grazing animals is met by the use of a dietary supplement.

This supplementation may be in the form of oral drenching with solid or liquid compositions, or by intramuscular injections.  These traditional methods of administration have suffered from various disadvantages. Liquid compositions, such as for example solutions of soluble salts of copper in the case of copper deficiency, are readily administered but are not retained in the animal and have to be given at very frequent intervals with attendant high labour costs of mustering and dosing. Intramuscular injections are limited to soluble forms of trace elements and typically special organic complexes of the trace elements are required. Even with organic complexes repeated injections are usually necessary. In addition tissue reactions frequently occur at the site of the injection and there is a high risk of acute general toxicity.

These problems have been particularly acute with the treatment of copper, cobalt, and selenium deficiency and many attempts have been made to develop solid materials comprising the desired trace element which will remain for long periods in the stomachs of the ruminant animal and steadily release the trace element for absorption in the alimentary tract. A particular difficulty in developing such solid materials has arisen from the nature of the digestive system of the ruminant animal.

To ensure that the trace element is not rapidly excreted it is necessary to use insoluble forms of the trace element and one approach has been to use the metallic or oxide forms of the trace elements. These forms can undergo slow dissolution in the rumen or abomasum of the ruminant animal and thus become available to the animal. Unfortunately the conditions in the rumen are such that some metals, particularly cobalt and copper, rapidly become coated with a phosphate or sulphide layer which prevents further dissolution of the trace element. Ideally the solid material would not remain in the rumen but would rapidly pass to the abomasum where the acid conditions
are more suitable for dissolution of metals and
metal oxides.   This selective deposition in the
abomasum is difficult to achieve since heavy particles
will remain in the bottom of the rumen and not reach
the abomasum, whereas lighter particles which can
transfer readily from the rumen to the abomasum are
also readily lost from the abomasum.   One approach that
has been tried is to use a relatively large solid
pellet or plug of trace element material in combination
with a "grinder", typically a grub screw, so that
although the solid pellet is retained in the rumen,
the action of the grinder is to constantly remove the
phosphate or sulphide coatings on the plug thus exposing
fresh surface of the plug for dissolution and absorption.
This approach has been used to some extent for treating
cobalt deficiency but has not been widely used with
other elements.   In particular, the method has not
been successful with copper because of the greater
susceptibility of that element to sulphide attack in
the rumen.   A further disadvantage of this method is
that both the pellets and grinders are hard and can
seriously cut and damage the mouth and soft tissue
parts of the animal.   Various devices have been used
to try to place the pellets as near the rumen as is
possible.   Thus there is a need for better methods
of treatment of trace element deficiency in ruminant
animals.

Australian Patent No 520489 discloses the use
of a particular copper-releasing substance, hereinafter
referred to as copper oxide needles, for the treatment
of copper deficiency in ruminant animals.   In this
disclosure the particles containing the trace element
have a high specific gravity and low mass such that
the particles are readily transferred from the rumen
but a high proportion are retained in the abomasum to

- 4 -          0159602

enable partial dissolution and absorption to take place over an extended period of time.

While this method has the potential to overcome some of the difficulties of earlier methods of treatment of trace element deficiencies, it has particular difficulties of its own related to the method of administration to the animals per os. In particular, the elongate particles of trace element material adapted to move readily from the rumen while remaining in the abomasum, are very fragile. This is particularly so in the case of the copper oxide needles referred to above. It has proved very difficult if not impossible to dose ruminant animals without damage to the elongate particles or needles occurring either by initial chewing by the ruminant animals, or by chewing following regurgitation of the particles or needles and the fragmented particles or needles do not have the ability to be retained in the abomasum and are therefore wastefully excreted. Various forms of pastes or gelatin capsules have been tried without success.

We have now discovered a novel means whereby particles of trace element materials may be satisfactorily used in the treatment of ruminant animals.

Accordingly we provide a therapeutic composition for the treatment of trace element deficiency in ruminant animals wherein said composition comprises a particulate source of at least one trace element suspended in a matrix of a solid polyethylene glycol.

In practice it has been found to be preferable, particularly in treatment of copper deficiency, that the particles of trace element are of a size, shape and density such that a substantial proportion will not be retained in the rumen for a period exceeding the usual period of retention of the normal contents of the rumen, while the particles will be retained in the abomasum substantially longer than the retention period for the normal contents of the abomasum. While the particles may be in any form, such as for example, spherical or irregular, a preferred particulate form is elongate or needle-like. The actual size, shape and density will vary to some extent with the particular trace element material.

Compositions of this type are particularly useful for the treatment of copper deficiency. The particulate form of copper preferred for the use of these compositions in treating copper deficiency is copper oxide needles which comprise copper coated with an oxide or oxides of copper. Preferably the copper oxide needles have a size or maximum dimension in the range of from 0.5 to 5.0 millimetres, and a relatively high specific gravity, for example, greater than 2.0 and most preferably greater than 5.0.

The polyethylene glycol used in the compositions of the present invention is preferably in the molecular weight range of 6000-20000 mass units, and preferably in the range of 8000-14000 mass units. Such polyethylene glycols are commonly referred to by those skilled in the art with an abbreviation, for example PEG 12000 is a reference to a polyethylene glycol of average molecular weight 12000. PEG 12000 is a preferred glycol for use in the compositions of our invention.

The idea of enclosing trace element material in a matrix was referred to in Australian Patent No. 520489 but it has not been possible hitherto to prepare a matrix which was both capable of effective administration and efficacious in the treatment of the trace element deficiency. The matrix material must be capable of breaking down in the rumen to release the trace element material and it must be free of toxicological side effects. There must be in addition a convenient method of combining the matrix and trace element materials into a suitable pellet. Many potential water-soluble matrix materials, for example inorganic salts, are relatively hard. If mixtures of these matrix materials and the trace element materials are pressed to form a pellet which is strong enough to withstand handling and administration to ruminants, the hard matrix materials cause considerable fragmentation of the trace element material. This is a particularly serious problem when the trace element material comprises copper oxide needles. Thus it has been accepted that pellets need to be formed by methods which do not involve pressing or the application of force to the fragile trace element material. Cement-type matrixes have been tried without success and gelatin capsules are also unsatisfactory.

It is a particular feature of our invention that the compositions comprise a matrix material that is nontoxic to ruminant animals and that is readily dissolved in the contents of the rumen to release the trace element material; that the compositions can be prepared in the form of a hard pellet that is easy to handle and administer; and furthermore that the pellets can be made without significant damage to even very fragile trace element material such as the copper oxide needles referred to hereinbefore.

In a further embodiment of our invention we provide a process of preparing a pellet of the compositions of our invention by pressing a mixture of polyethylene glycol and trace element material with a force in the range of 1 to 25 tonnes, and preferably in the range of 1 to 10 tonnes. Forces in excess of 25 tonnes can be used but damage to fragile trace element material, in particular copper oxide needles, can occur.

A surprising and particularly advantageous feature of the compositions prepared by the process of the invention is that the particulate matter tends to concentrate in the centre of the pellet. This feature is surprising as it has been found that if carriers other than polyethylene glycols are used to form pellets the particulate matter frequently concentrates at an outer surface of the pellet, for example at the bottom of the die, which often results in damage to fragile particulate matter and unsatisfactory pellets. This feature is particularly advantageous as concentration of the particulate matter in the centre of the pellet reduces damage to the particulate material during the formation of the pellet. Moreover, concentration of the particulate matter in the centre of the pellet means that the pellets increase in specific gravity in the rumen as the polyethylene glycol dissolves, further reducing the likelihood of regurgitation and mastication or expectoration of the pellet.

Although it is not intended that the invention be bound by theory it is believed that the particle size of the polyethylene glycol may influence the formation of pellets according to the process of the invention. In practice, it has been found preferable to use polyethylene glycol of particle size in the range of from 200 $\mu$ m to 1200 $\mu$ m and more preferably the particle size in the range from 300 $\mu$ m to 900 $\mu$ m.

The ratio of polyethylene glycol to trace element material is important. For example, with copper oxide needles, the weight/weight ratio of polyethylene glycol to copper oxide needles is preferably at least 1.4:1. The upper limit of the ratio of polyethylene glycol to copper oxide needles is not critical as far as the damage to the needles or the efficacy in the ruminant animal is concerned, but too large an excess of polyethylene glycol is wasteful and an increase in the size of the pellet (for a given dose size of trace element material) can create difficulties in oral administration of animals.

Pellets of polyethylene glycol and copper oxide needles prepared under the preferred conditions, and with the ratios and particle sizes referred to hereinbefore, provide hard pellets which can be dropped onto hard concrete floors from a height of 1.5 metres without damage.  This hardness is more than adequate for all normal handling conditions including the use of conventional pellet dispensing guns.

The aspect ratio or shape of the pellet is not narrowly critical but no dimension should be smaller than the length of the elongate particles or needles used, since in that case breakage will occur. Preferably the size of the pellet will be such as to be readily administerable by a device such as the conventional pellet gun.  The die used to press the pellet is conveniently of cylindrical cross-section since this type is readily available. The faces of the upper punch and lower die block or punch may be bevelled or hemispherical.  The pellets may be tumbled briefly to remove sharp edges but this is not essential since the compositions of our invention cause no tissue damage in contrast to many of the prior art pellets or boluses.  Typical pellets of the compositions of our invention used for the treatment of copper deficiency in sheep are short cylinders of ca 12-14 mm diameter and 15-20 mm length.  Typical pellets of the composition of our invention used for the treat- of copper deficiency in cattle are cylinders of ca 17-20 mm diameter and 20-25 mm length.

The dose rate for treating ruminants for trace element deficiency depends on the particular trace element material and is also proportional to the body weight of the animal.  An optimal size of pellet or pellets can be readily determined for each particular application by those skilled in the art.

For treatment of copper deficiency in sheep with copper oxide needles, for example, a dose of 1.25 g is satisfactory for animals with a body weight up to 20 kg, and 2.5 g is satisfactory for animals in excess of 35 20 kg.  It is convenient therefore, for this application, to prepare pellets each containing 1.25 g of copper oxide needles so that the dosing regimen is reduced to a choice of one or two pellets.  Typical pellets of copper oxide needles in polyethylene glycol prepared by our process have diameters of ca 12 mm and lengths of 16 mm.  These can be readily administered by existing commercial pellet dispensing guns.

The compositions of our invention may comprise more than one trace element material where it is desired to correct more than one trace element deficiency.  In a further embodiment of the process of our invention we provide a method of treating trace element deficiency in ruminant animals by the oral administration of pellets prepared by pressing a mixture comprising a polyethylene glycol in the molecular weight range of from 6000 to 20000 mass units, preferably in the range of from 8000 to 14000 and a particulate trace element material with a force in the range of from 1 to 25 tonnes.

In a yet further embodiment of our invention we provide compositions, processes, and methods of treatment as hereinbefore described, wherein the compositions in addition comprise therapeutic agents in the matrix for the control of endoparasitic infections in the animals being treated for trace element deficiency.

By endoparasitic infections we mean gastro-intestinal and pulmonary nematodes such as, for example, Haemonchus spp, Ostertagia spp, Trichostronglus spp, Nematodirus spp, Oesophagostomum spp, Chabertia spp, and Dictyocaulus spp and liver fluke such as, for example Fasciolia spp.

Suitable therapeutic agents for the control of gastro-intestinal and pulmonary nematodes include, for example, thiabendazole, pyrantel, parbendazole, cambendazole, mebendazole, oxibendazole, fenbendazole, albendazole, oxfendazole, morantel, oxyclozanide, febantel and tetramisole.

A particularly preferred therapeutic agent for inclusion in the compositions of our invention is the active isomer of tetramisole, namely levamisole. Tetramisole is the common name for the hydrochloride salt of the anthelmintic dl-2,3,5,6tetrahydro-imidazo [2,3-b] thiazole, and levamisole is the common name for the laevorotatory isomer of tetramisole.

This invention is now illustrated by but not limited to the following examples in which all parts and percentages are by weight unless otherwise specified.

Example 1

The pellets containing the copper oxide needles (CON) are prepared by the following general procedure. A mixture of polyethylene glycol and CON is blended and poured into a stainless steel die. The diameter of the pellet is determined by the diameter of the die and the length of the pellet is determined by the total quantity of polyethylene glycol and CON used. The shape of the end of the pellet is determined by the shape of the punches placed in contact with the said mixture at either end of the die cavity. The surface of these inserts in contact with the mixture are conveniently flat, but may be concave or bevelled.

The mixture is then subject to compression. A compression load of at least one tonne is required to generate a pellet which will not shatter when dropped three times in succession from a height of 1.5 metres onto a rigid surface such as a concrete floor. This is regarded as the minimum specification for pellet hardness for satisfactory handling of the pellet from all stages from manufacture through to administration and ingestion into the rumen of the treated animals. Compression loads of 1 to 25 tonnes have been used successfully but needle breakage tends to occur near the upper limit and a range of 1 to 10 tonnes is more commonly used.

Example 2

Six 6 month old merino first-cross wethers weighing between 35-40 kg were each dosed with 3 pellets. Each pellet contained CON (1.25 g) and PEG 12000 (1.85 g) and had been pressed at 7 tonnes. The diameter and length of the pellet after compression were 12.7 mm and 16.0 $\pm$ 0.2 mm respectively.

The sheep were allowed to stand on a cleaned concrete floor and monitored for regurgitation and expectoration for 2 hours. No pellets were expectorated during this time.

Example 3

Pellets were prepared by compressing at 15 tonnes a mixture of CON (1.25 g), PEG 12000 (1.85 g) and BRILLIANT FCF C.I. No 42090 (2 mg). The diameter and length of the pellets were 14.0 mm and 19.4 $\pm$ 0.3 mm respectively. Eight cross-bred merino wethers (ca 50 kg each) were each dosed orally with 2 pellets and then allowed to stand on a concrete floor for observation. Over a period of two hours no expectoration of pellets was noted and there was no colouring of the mouth or throat areas with blue dye from the pellets indicating that no regurgitation of the pellets had occurred.

Example 4

The procedure of Example 3 was repeated on one cross-bred merino wether except that 4 pellets were administered. There was no regurgitation of the pellets or dyeing of the mouth and throat areas over a period of two hours, at which stage the animal was sacrificed. No trace of the original pellets could be found in the rumen although there were distinct blue en where the pellets had pellet had contained ca 72 copper oxide needles, a total dose of 288 needles. On examination of the sacrificed animal 232 needles were recovered, 196 in the rumen and 36 in the abomsum. No needles were found in the small intestine.

## Example 5

Pellets of length 23 mm and diameter 19 mm suitable for use in cattle may be prepared by compressing at 15 tonnes a mixture of CON (4.0 g), PEG 1200 (6 g) and BRILLIANT FCF C.I. No 42090 (5 mg). Hereford stears may be dosed with such pellets without sign of expectoration or regurgitation.

## Example 6

Mature cross-bred wethers (ca 55 g) were fistulated into the rumen. The pellets used in this example contained CON (1.25 g) and PEG 12000 (2.75 g) and were pressed with a force of 15 tonnes to yield pellets of diameter 14.0 mm and length $19.4 \pm 0.3$ mm.

Six pellets were placed into separate nylon bags weighted down with glass beads, and having attached a nylon line of ca one metre in length. Each bag was placed into a fistulated animal at the start of the experiment and allowed to settle in the rumen fluid. One bag containing pellets was removed at each 10 minute interval from the start and the pellet residues removed from the bag and allowed to dry. The weight and specific gravity of each pellet was then determined and average weights and specific gravities at each time interval calculated. The results are shown in Table 1. Qualitative observation indicated that the copper oxide needles had been concentrated in the centre of the pellet by the pressing process and this was consistent with the rise in specific gravity of the pellets in the early period in the rumen. This increase in specific gravity is beneficial in further reducing the likelihood of regurgitation of the pellet with consequent needle damage.

TABLE 1

| TIME | INITIAL PELLET WEIGHT | FINAL PELLET WEIGHT | PELLET DENSITY | WT OF NEEDLES LIBERATED |
|------|-----------------------|---------------------|----------------|-------------------------|
| (MIN) | (GM) | (GM) | (GM/ML) | (GM) |
| 0 | – | – | 1.1 | – |
| 10 | 4.096 | 3.237 | 1.9 | 0 |
| 20 | 4.071 | 2.413 | 2.0 | .0478 |
| 30 | 3.948 | 2.050 | 2.1 | .1000 |
| 40 | 3.787 | 1.665 | 2.7 | .1396 |
| 50 | 4.014 | 1.055 | 2.9 | .4915 |
| 60 | 4.068 | .552 | 2.8 | .6269 |

Example 7

A pellet was prepared by the procedure of Example 1 using a mixture of PEG 12000 (2.6g), CON (1.25g) and the free base of levamisole (0.15g) and pressing with a force of 15 tonnes. The pellets had diameters of 14.0 mm and lengths of 19.4 ± 0.3 mm. No significant damage to the copper oxide needles occurred during the pressing and the pellets withstood the dropping test referred to in Example 1.

Example 8

The procedure of Example 6 was repeated except that the free base of levamisole was replaced by an equal weight of levamisole in the form of the hydrochloride salt. The pellets were of the same size as the pellets produced by the method of Example 6. No significant fragmentation of the needles occurred during pressing the pellets survived the usual dropping test.

Example 9

Three year old first-cross merino ewes (144) suffering from copper deficiency were kept in holding yards overnight with access to food and water. The following day they were placed in pens 1.5 hours before dosing with CON pellets. Each animal was dosed with 2 pellets from a commercial pellet gun. Each pellet contained CON (1.25g) and PEG 12000 (1.85g) and had been pressed at 1 tonne to produce a diameter of 12.3 mm and a length of 17.0 ± 0.3 mm. The animals were held in the pens for 1.5 hours after dosing for observation, and no regurgitation of pellets was noted.

Example 10

Pellets were prepared according to the procedure of Example 1 using polyethylene glycols having a range of molecular weights and particle sizes. Pellets having the most desirable properties were obtained using polyethylene glycols having a molecular weight in the range of 6 000 to 20 000 mass units and a particle size in the range from 200 $\mu$ m to 1200 $\mu$ m and preferably 300 $\mu$ m to 900 $\mu$ m.

Comparative Example 1

This is a comparative example of a paste formulation of CON used to treat copper-deficient ruminants.

First-cross merinos (ca 30 kg) each received 2 ml (equivalent to 2.5g CON) of the following paste formulations from a commercial veterinary paste gun.

Formulation No 1

CON (30g) in 19 ml of a paste prepared from Attagel 40 (50g) and distilled water.

Formulation No 2

CON (30g) in 19 ml of a paste prepared from Volclay 325 (500g) and distilled water (200 ml).
Formulation No 3

CON (30g) in 19 ml of a paste prepared from PEG 300 (50g) and PEG 1500 (50g).

In all these cases, although the paste was swallowed readily, the copper oxide needles were chewed and seriously fragmented.

- 17 -

CLAIMS

1.    A therapeutic composition for the treatment of trace element deficiency in ruminant animals wherein said composition comprises a particulate source of at least one trace element suspended in a matrix of solid polyethylene glycol.

2.    A therapeutic composition according to claim 1 wherein said trace element is copper.

3.    A therapeutic composition according to claim 1 or claim 2 wherein said trace element is copper in the form of copper oxide needles comprising copper coated with an oxide or oxides of copper.

4.    A composition according to claim 3 wherein the weight/weight ratio of polyethylene glycol to copper oxide needles is at least 1.4:1.

5.    A therapeutic composition according to claim 3 or claim 4 wherein copper oxide needles have a size or maximum dimension in the range from 0.5 to 5.0 mm and a specific gravity of greater than 2.0.

6.      A therapeutic compositon according to any one
of claims 1 to 5 inclusive wherein said polyethylene
glycol has a molecular weight in the range of from
6 000 to 20 000.

7.      A composition according to any one of claims 1
to 6 inclusive wherein the particle size of polyethylene glycol is in the range of from 200  $\mu$ m to
1200 $\mu$ m.

8.      A composition according to any one of claims
1 to 7 inclusive wherein said composition is in the
form of pellets.

9.      A composition according to claim 8 wherein
said pellets are in the form of cylinders of diameter
in the range 12-14 mm and length in the range 15-20
mm.

10.     A composition according to claim 8 or claim
9 inclusive wherein each pellet contains 1.25 g of
copper oxide needles comprising copper coated
with an oxide or oxides of copper.

11.     A composition according to claim 8 wherein
said pellets are in the form of cylinders of diameter
in the range 18-20 mm diameter and length in the
range 20-25 mm.

12.     A composition according to any one of claims
1 to 11 inclusive wherein said composition further
comprises at least one therapeutic agent for the
control of endoparasitic infections.

0159602

13.     A composition according to claim 12 wherein said therapeutic agent is levamisole.

14.     A process for the preparation of pellets according to any one of claims 8 to 11 inclusive which process comprises pressing a mixture of polyethylene glycol and trace element material with a force in the range of 1 to 25 tonnes.

15.     A method of treating trace element deficiency in ruminant animals which process comprises oral administration to said animals of a composition according to any one of claims 1 to 13.

16.     A method according to claim 15 wherein said animals are sheep or cattle.

17.     A composition as defined according to any one of claims 1 to 13 as herein described with reference to any one of Examples 1 to 10 inclusive.

18.     A process for the preparation of pellets according to claim 14 and as herein described with reference to Example 1 or Example 2.

19.     A method of treatment according to claim 15 or claim 16 as herein described with reference to any one of Examples 2, 3, 4, 5, 6 and 9.

DATED this                day of                1985

ICI AUSTRALIA LIMITED